# EUROPEAN PATENT SPECIFICATION

(11) **EP 2 057 899 B1**
(45) Date of publication and mention of the grant of the patent: **01.09.2010**
(21) Application number: 08166736.2
(22) Date of filing: 16.10.2008
(51) Int. Cl.: A01N 27/00

(54) **Method for making cyclopropene inclusion complex**
Verfahren zur Herstellung von Cyclopropen-Einschlusskomplexen
Procédé pour la fabrication d'un complexe d'inclusion de cyclopropène

(30) Priority: 06.11.2007 US 1991 P
(43) Date of publication of application: 13.05.2009
(60) Divisional application: 10150976.8
(73) Proprietor: Rohm and Haas Company, Philadelphia, PA 19106-2399 (US)
(72) Inventor: Jacobson, Richard Martin, Chalfont, PA 18914 (US)
(74) Representative: Kent, Venetia Katherine

(56) References cited:
- WO-A-00/10386
- US-A1- 2002 043 730
- SABADINI, E. ET AL.: "Solubility of cyclomaltooligosaccharides (cyclodextrins) in H2O and D2O: a comparative study" CARBOHYDRATE RESEARCH, vol. 341, no. 1, 16 January 2006 (2006-01-16), pages 270-274, XP002518396
- FOURMENTIN S. ET AL.: "Cyclodextrins: a potential absorbent for VOC abatement" GLOBAL NEST, vol. 8, no. 3, 2006, pages 324-329, XP002518394 Greece
- TZE LOON NEOH, ET AL: "Kinetics of Molecular Encapsulation of 1-Methylcyclopropene into Alpha-Cyclodextrin" J. AGRIC. FOOD CHEM., vol. 55, no. 26, 4 December 2007 (2007-12-04), pages 11020-11026, XP002518395

## Description

### BACKGROUND:

It is often desired to make cyclopropene complex that contains cyclopropene and molecular encapsulating agent.

In the past, one approach to making such complexes involved batch methods in which cyclopropene was added to a fixed amount of a mixture of molecular encapsulating agent and solvent. For example, US 6,313,068 describes placing alpha-cyclodextrin, buffer solution, and 1-methylcyclopropene into a mixing vessel, sealing the mixing vessel (i.e., closing all connections to the mixing vessel), placing the mixing vessel on a shaker, removing the contents from the mixing vessel, and then separating the cyclopropene complex from the other contents of the mixing vessel by filtration. It is desired to provide a batch method of making cyclopropene complex that can be performed in an un-sealed vessel. It is also desired to provide such a batch method that also has one or more of these additional characteristics: that employs high concentration of molecular encapsulating agent; that produces high yield of cyclopropene complex; that minimizes waste water, that minimizes waste of molecular encapsulating agent; and/or that minimizes waste of cyclopropene complex.

Another approach that was used in the past for making cyclopropene complexes was the use of a continuous process. For example, US 6,953,540 describes bubbling 1-methcylcyclopropene gas into a stirred vessel containing a solution of alpha-cyclodextrin. In the procedure described by US 6,953,540, fresh solution of alpha-cyclodextrin was added continuously as 1-methylcyclopropene was also continuously added, and cyclopropene complex was continuously removed.

### STATEMENT OF THE INVENTION:

In the present invention, there is provided a batch method for making cyclopropene complex comprising
(a) forming a mixture in a vessel, said mixture comprising
   (i) one or more molecular encapsulating agent and
   (ii) solvent,
   wherein the ratio of the amount of said (i) to the amount of said (ii) is higher than the solubility of said molecular encapsulating agent in said solvent, and
(b) introducing one or more cyclopropene into said mixture to form said cyclopropene complex, and
(c) after said step (a) and said step (b), removing some or all of said cyclopropene complex from said vessel,
wherein said vessel is not sealed while said method is being performed.

### DETAILED DESCRIPTION:

The practice of the present invention involves the use of one or more cyclopropene. As used herein, "a cyclopropene" is any compound with the formula 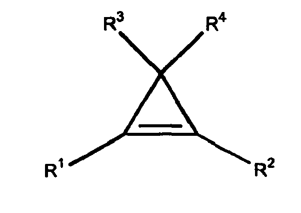 where each R¹, R², R³ and R⁴ is independently selected from the group consisting of H and a chemical group of the formula:

-(L)ₙ-Z

where n is an integer from 0 to 12. Each L is a bivalent radical. Suitable L groups include, for example, radicals containing one or more atoms selected from H, B, C, N, O, P, S, Si, or mixtures thereof. The atoms within an L group may be connected to each other by single bonds, double bonds, triple bonds, or mixtures thereof. Each L group may be linear, branched, cyclic, or a combination thereof. In any one R group (i.e., any one of R¹, R², R³ and R⁴) the total number of heteroatoms (i.e., atoms that are neither H nor C) is from 0 to 6. Independently, in any one R group the total number of non-hydrogen atoms is 50 or less. Each Z is a monovalent radical. Each Z is independently selected from the group consisting of hydrogen, halo, cyano, nitro, nitroso, azido, chlorate, bromate, iodate, isocyanato, isocyanido, isothiocyanato, pentafluorothio, and a chemical group G, wherein G is a 3 to 14 membered ring system.

The R¹, R², R³, and R⁴ groups are independently selected from the suitable groups. The R¹, R², R³, and R⁴ groups may be the same as each other, or any number of them may be different from the others. Among the groups that are suitable for use as one or more of R¹, R², R³, and R⁴ are, for example, aliphatic groups, aliphatic-oxy groups, alkylphosphonato groups, cycloaliphatic groups, cycloalkylsulfonyl groups, cycloalkylamino groups, heterocyclic groups, aryl groups, heteroaryl groups, halogens, silyl groups, other groups, and mixtures and combinations thereof. Groups that are suitable for use as one or more of R¹, R², R³, and R⁴ may be substituted or unsubstituted. Independently, groups that are suitable for use as one or more of R¹, R², R³, and R⁴ may be connected directly to the cyclopropene ring or may be connected to the cyclopropene ring through an intervening group such as, for example, a heteroatom-containing group.

Among the suitable R¹, R², R³, and R⁴ groups are, for example, aliphatic groups. Some suitable aliphatic groups include, for example, alkyl, alkenyl, and alkynyl groups. Suitable aliphatic groups may be linear, branched, cyclic, or a combination thereof. Independently, suitable aliphatic groups may be substituted or unsubstituted.

As used herein, a chemical group of interest is said to be "substituted" if one or more hydrogen atoms of the chemical group of interest is replaced by a substituent. It is contemplated that such substituted groups may be made by any method, including but not limited to making the unsubstituted form of the chemical group of interest and then performing a substitution. Suitable substituents include, for example, alkyl, alkenyl, acetylamino, alkoxy, alkoxyalkoxy, alkoxycarbonyl, alkoxyimio, carboxy, halo, haloalkoxy, hydroxy, alkylsulfonyl, alkylthio, trialkylsilyl, dialkylamino, and combinations thereof. An additional suitable substituent, which, if present, may be present alone or in combination with another suitable substituent, is

-(L)ₘ-Z

where m is 0 to 8, and where L and Z are defined herein above. If more than one substituent is present on a single chemical group of interest, each substituent may replace a different hydrogen atom, or one substituent may be attached to another substituent, which in turn is attached to the chemical group of interest, or a combination thereof.

Among the suitable R¹, R², R³, and R⁴ groups are, for example, substituted and unsubstituted aliphatic-oxy groups, such as, for example, alkenoxy, alkoxy, alkynoxy, and alkoxycarbonyloxy.

Also among the suitable R¹, R², R³, and R⁴ groups are, for example, substituted and unsubstituted alkylphosphonato, substituted and unsubstituted alkylphosphato, substituted and unsubstituted alkylamino, substituted and unsubstituted alkylsulfonyl, substituted and unsubstituted alkylcarbonyl, and substituted and unsubstituted alkylaminosulfonyl, including, for example, alkylphosphonato, dialkylphosphato, dialkylthiophosphato, dialkylamino, alkylcarbonyl, and dialkylaminosulfonyl.

Also among the suitable R¹, R², R³, and R⁴ groups are, for example, substituted and unsubstituted cycloalkylsulfonyl groups and cycloalkylamino groups, such as, for example, dicycloalkylaminosulfonyl and dicycloalkylamino.

Also among the suitable R¹, R², R³, and R⁴ groups are, for example, substituted and unsubstituted heterocyclyl groups (i.e., aromatic or non-aromatic cyclic groups with at least one heteroatom in the ring).

Also among the suitable R¹, R², R³, and R⁴ groups are, for example, substituted and unsubstituted heterocyclyl groups that are connected to the cyclopropene compound through an intervening oxy group, amino group, carbonyl group, or sulfonyl group; examples of such R¹, R², R³, and R⁴ groups are heterocyclyloxy, heterocyclylcarbonyl, diheterocyclylamino, and diheterocyclylaminosulfonyl.

Also among the suitable R¹, R², R³, and R⁴ groups are, for example, substituted and unsubstituted aryl groups. Suitable substituents are those described herein above. In some embodiments, one or more substituted aryl group is used in which at least one substituent is one or more of alkenyl, alkyl, alkynyl, acetylamino, alkoxyalkoxy, alkoxy, alkoxycarbonyl, carbonyl, alkylcarbonyloxy, carboxy, arylamino, haloalkoxy, halo, hydroxy, trialkylsilyl, dialkylamino, alkylsulfonyl, sulfonylalkyl, alkylthio, thioalkyl, arylaminosulfonyl, and haloalkylthio.

Also among the suitable R¹, R², R³, and R⁴ groups are, for example, substituted and unsubstituted heterocyclic groups that are connected to the cyclopropene compound through an intervening oxy group, amino group, carbonyl group, sulfonyl group, thioalkyl group, or aminosulfonyl group; examples of such R¹, R², R³, and R⁴ groups are diheteroarylamino, heteroarylthioalkyl, and diheteroarylaminosulfonyl.

Also among the suitable R¹, R², R³, and R⁴ groups are, for example, hydrogen, fluoro, chloro, bromo, iodo, cyano, nitro, nitroso, azido, chlorato, bromato, iodato, isocyanato, isocyanido, isothiocyanato, pentafluorothio; acetoxy, carboethoxy, cyanato, nitrato, nitrito, perchlorato, allenyl; butylmercapto, diethylphosphonato, dimethylphenylsilyl, isoquinolyl, mercapto, naphthyl, phenoxy, phenyl, piperidino, pyridyl, quinolyl, triethylsilyl, trimethylsilyl; and substituted analogs thereof.

As used herein, the chemical group G is a 3 to 14 membered ring system. Ring systems suitable as chemical group G may be substituted or unsubstituted; they may be aromatic (including, for example, phenyl and napthyl) or aliphatic (including unsaturated aliphatic, partially saturated aliphatic, or saturated aliphatic); and they may be carbocyclic or heterocyclic. Among heterocyclic G groups, some suitable heteroatoms are, for example, nitrogen, sulfur, oxygen, and combinations thereof. Ring sysytems suitable as chemical group G may be monocyclic, bicyclic, tricyclic, polycyclic, spiro, or fused; among suitable chemical group G ring systems that are bicyclic, tricyclic, or fused, the various rings in a single chemical group G may be all the same type or may be of two or more types (for example, an aromatic ring may be fused with an aliphatic ring).

In some embodiments, G is a ring system that contains a saturated or unsaturated 3 membered ring, such as, for example, a substituted or unsubstituted cyclopropane, cyclopropene, epoxide, or aziridine ring.

In some embodiments, G is a ring system that contains a 4 membered heterocyclic ring; in some of such embodiments, the heterocyclic ring contains exactly one heteroatom. Independently, in some embodiments, G is a ring system that contains a heterocyclic ring with 5 or more members; in some of such embodiments, the heterocyclic ring contains 1 to 4 heteroatoms. Independently, in some embodiments, the ring in G is unsubstituted; in other embodiments, the ring system contains 1 to 5 substituents; in some of the embodiments in which G contains substituents, each substituent is independently chosen from the substituents described herein above. Also suitable are embodiments in which G is a carbocyclic ring system.

In some embodiments, each G is independently a substituted or unsubstituted phenyl, pyridyl, cyclohexyl, cyclopentyl, cycloheptyl, pyrolyl, furyl, thiophenyl, triazolyl, pyrazolyl, 1,3-dioxolanyl, or morpholinyl. Among these embodiments include those embodiments, for example, in which G is unsubstituted or substituted phenyl, cyclopentyl, cycloheptyl, or cyclohexyl. In some of these embodiments, G is cyclopentyl, cycloheptyl, cyclohexyl, phenyl, or substituted phenyl. Among embodiments in which G is substituted phenyl are embodiments, for example, in which there are 1, 2, or 3 substituents. Independently, also among embodiments in which G is substituted phenyl are embodiments, for example, in which the substituents are independently selected from methyl, methoxy, and halo.

Also contemplated are embodiments in which R³ and R⁴ are combined into a single group, which is attached to the number 3 carbon atom of the cyclopropene ring by a double bond. Some of such compounds are described in US Patent Publication 2005/0288189.

In some embodiments, one or more cyclopropenes are used in which one or more of R¹, R², R³, and R⁴ is hydrogen. In some embodiments, R¹ or R² or both R¹ and R² is hydrogen. Independently, in some embodiments, R³ or R⁴ or both R³ and R⁴ is hydrogen. In some embodiments, R², R³, and R⁴ are hydrogen.

In some embodiments, one or more of R¹, R², R³, and R⁴ is a structure that has no double bond. Independently, in some embodiments, one or more of R¹, R², R³, and R⁴ is a structure that has no triple bond. Independently, in some embodiments, one or more of R¹, R², R³, and R⁴ is a structure that has no halogen atom substituent. Independently, in some embodiments, one or more of R¹, R², R³, and R⁴ is a structure that has no substituent that is ionic.

In some embodiments, one or more of R¹, R², R³, and R⁴ is hydrogen or (C₁-C₁₀) alkyl. In some embodiments, each of R¹, R², R³, and R⁴ is hydrogen or (C₁-C₈) alkyl. In some embodiments, each of R¹, R², R³, and R⁴ is hydrogen or (C₁-C₄) alkyl. In some embodiments, each of R¹, R², R³, and R⁴ is hydrogen or methyl. In some embodiments, R¹ is (C₁-C₄) alkyl and each of R², R³, and R⁴ is hydrogen. In some embodiments, R¹ is methyl and each of R², R³, and R⁴ is hydrogen, and the cyclopropene is known herein as 1-methylcyclopropene ("1-MCP").

In some embodiments, a cyclopropene is used that has boiling point at one atmosphere pressure of 50°C or lower; or 25°C or lower; or 15°C or lower. Independently, in some embodiments, a cyclopropene is used that has boiling point at one atmosphere pressure of -100°C or higher; -50°C or higher; or -25°C or higher; or 0°C or higher.

The practice of the present invention involves the use of one or more molecular encapsulating agent. Suitable molecular encapsulating agents include, for example, organic and inorganic molecular encapsulating agents. Suitable organic molecular encapsulating agents include, for example, substituted cyclodextrins, unsubstituted cyclodextrins, and crown ethers. Suitable inorganic molecular encapsulating agents include, for example, zeolites. The preferred molecular encapsulating agent will vary depending upon the structure of the cyclopropene or cyclopropenes being used.

In some embodiments, the molecular encapsulating agent contains one or more cyclodextrin. In some embodiments, every molecular encapsulating agent that is used is a cyclodextrin.

Cyclodextrins are compounds whose molecules are cone-shaped structures that have structures that are made from 6 or more glucose units. As used herein, a statement that a cyclodextrin is made from certain glucose units is to be understood as a description of the structure of the cyclodextrin molecule, which may or may not be actually made by reacting those certain glucose molecules. Cyclodextrins may be made from as many as 32 glucose units. Cyclodextrins that are made from 6, 7, and 8 glucose units are known, respectively, as alpha-cyclodextrin, beta-cyclodextrin, and gamma-cyclodextrin. Some cyclodextrins are available, for example, from Wacker Biochem Zinc., Adrian, MI, as well as other vendors.

Independent of the number of glucose units in the cyclodextrin, the class of compounds called "cyclodextrins" is considered herein to also include derivatives of cyclodextrin molecules. That is, the term "cyclodextrin" applies herein to molecules that are cone-shaped structures that have structures that are made from 6 or more glucose units and also applies to derivatives of such molecules, when such derivatives are capable of performing as molecular encapsulating agents. Some suitable derivatives are, for example, molecules that have a structure that is (or could be) formed by addition of an alkyl group (such as, for example, a methyl group) to a cyclodextrin. Some other derivatives are, for example, molecules that have a structure that is (or could be) formed by addition of a hydroxyalkyl group (such as, for example, a hydroxypropyl group) to a cyclodextrin. Some derivatives that are considered "cyclodextrins" are, for example, methyl-beta-cyclodextrin and hydroxypropyl-alpha-cyclodextrin.

In some embodiments, exactly one of alpha-cyclodextrin, beta-cyclodextrin, or gamma-cyclodextrin is used. In some embodiments, a mixture of two or more of alpha-cyclodextrin, beta-cyclodextrin, and gamma-cyclodextrin is used. In some embodiments, alpha-cyclodextrin is used. In some embodiments, no molecular encapsulating agent other than alpha-cyclodextrin is used.

Mixtures of suitable molecular encapsulating agents are also suitable.

The present invention involves one or more cyclopropene complex. A cyclopropene complex is a composition in which one or more molecular encapsulating agent encapsulates one or more cyclopropene or a portion of one or more cyclopropene.

In some embodiments, at least one cyclopropene complex is an inclusion complex. In such an inclusion complex, the molecular encapsulating agent forms a cavity, and the cyclopropene or a portion of the cyclopropene is located within that cavity. In some of such inclusion complexes, there is no covalent bonding between the cyclopropene and the molecular encapsulating agent. Independently, in some of such inclusion complexes, there is no ionic bonding between the cyclopropene and the molecular encapsulating complex, whether or not there is any electrostatic attraction between one or more polar moiety in the cyclopropene and one or more polar moiety in the molecular encapsulating agent.

Independently, in some of such inclusion complexes, the interior of the cavity of the molecular encapsulating agent is substantially apolar or hydrophobic or both, and the cyclopropene (or the portion of the cyclopropene located within that cavity) is also substantially apolar or hydrophobic or both. While the present invention is not limited to any particular theory or mechanism, it is contemplated that, in such apolar cyclopropene complexes, van der Waals forces, or hydrophobic interactions, or both, cause the cyclopropene molecule or portion thereof to remain within the cavity of the molecular encapsulating agent.

The cyclopropene complex can usefully be characterized by the ratio of moles of molecular encapsulating agent to moles of cyclopropene. In some embodiments, the ratio of moles of molecular encapsulating agent to moles of cyclopropene is 0.1 or larger; or 0.2 or larger; or 0.5 or larger; or 0.9 or larger. Independently, in some of such embodiments, the ratio of moles of molecular encapsulating agent to moles of cyclopropene is 2 or lower; or 1.5 or lower.

The present invention involves the use of solvent. Any solvent is suitable that is capable of dissolving some of the encapsulating agent. In some embodiments, the amount of encapsulating agent that is soluble in the solvent is, by weight based on the weight of solvent, 0.1% or more; or 0.3% or more; or 1% or more; or 3% or more. Independently, in some embodiments, the amount of encapsulating agent that is soluble in the solvent is, by weight based on the weight of solvent, 50% or less; or 25% or less.

Some suitable solvents are nonpolar. Some suitable solvents are polar (that is, having dipole moment of 1.0 Debye units or higher). Independently, some suitable solvents are aqueous. An aqueous solvent is a solvent that contains 50% or more water by weight, based on the weight of solvent. In some embodiments, aqueous ' solvent is used that has an amount of water of 75% or more; or 85% or more; or 95% or more; by weight based on the weight of solvent. Independently, in some embodiments, a solvent is used that is not aqueous.

One characteristic of a molecular encapsulating agent is its solubility in a specific solvent at a given temperature. As used herein, that "solubility" is the maximum amount of the molecular encapsulating agent that can be dissolved in that solvent at a given temperature. As used herein, "solubility" refers to the solubility at 20°C unless otherwise stated. The solubility of a molecular encapsulating agent in a particular solvent is measured separately from performing a method of the present invention; that solubility is measured by examining experimental mixtures of pure (or as nearly pure as is commonly used in industrial processes) molecular encapsulating agent and solvent, without the presence of other ingredients in the experimental mixture. The solubility of a molecular encapsulating agent in a solvent is measured using molecular encapsulating agent that is not participating in an inclusion complex with cyclopropene or with any other material other than, possibly, the solvent.

Herein, the solubility of a molecular encapsulating agent in a particular embodiment refers to the solubility of that molecular encapsulating agent in the solvent used in that embodiment, at the temperature at which that embodiment is performed.

Some embodiments of the present invention involve the use of one or more cyclodextrin and the use of aqueous solvent. In such embodiments, one useful method of measuring the concentration of cyclodextrin in the solvent is the Brix method. The Brix method is usually performed by measuring the refractive index of a solution. The relationship of refractive index to sugar concentration is well known, for example in tables published by the International Commission for Uniform Methods of Sugar Analysis (ICUMSA). The results of the Brix method are reported as "degrees Brix," where X degrees Brix is the same as X% sugar by weight.

In some embodiments of the present invention, one or more cyclopropene complex is formed that has low solubility in the solvent. In some embodiments, the solubility of one or more cyclopropene complex formed by the method of the present invention is, by weight of cyclopropene complex based on the weight of solvent, 1% or less; or 0.5% or less; or 0.2% or less; or 0.1% or less.

The vessel in which formation of cyclopropene complex takes place is known herein as the "reactor."

The present invention involves making cyclopropene complex using a batch method. A batch method is a method in which, at some point in the method, addition of ingredients to the reactor is completed and product is subsequently removed from the reactor. In a batch method, ingredients may be added individually to the reactor, or a mixture of two or more ingredients may be added to the reactor. In a batch method, each ingredient, independent of other ingredients, may be added to the reactor suddenly or gradually or intermittently or a combination thereof. In a batch method, appropriate operations, such as, for example, agitation, adjustment of temperature, other operations, and combinations thereof, may be performed before, during, or after the addition of ingredients, or any combination thereof.

In performing batch embodiments of the present invention, solvent, one or more molecular encapsulating agent, one or more cyclopropene, and, optionally, one or more other ingredient are added to a reactor; at some point, addition of ingredients is completed, and subsequently cyclopropene complex is removed. In such embodiments, it is useful to characterize the result of the method by the molecular encapsulating agent yield, which is the ratio of the weight of molecular encapsulating agent that is removed from the reactor as part of a cyclopropene complex to the total weight of molecular encapsulating agent added to the reactor prior to removal of cyclopropene complex. In some embodiments, the molecular encapsulating agent yield is 50% or higher; or 70% or higher; or 90% or higher; or 95% or higher.

In batch methods of the present invention, it is also useful to characterize the concentration of molecular encapsulating agent in the reactor, which is the total weight of molecular encapsulating agent (that is, the total weight of all molecular encapsulating agent in the reactor that is added to the reactor prior to removal of cyclopropene complex) as a percentage based on the sum of the total weight of molecular encapsulating agent and total weight of solvent that is added to the reactor prior to removal of cyclopropene complex.

In each batch embodiment of the present invention, the molecular encapsulating agent concentration is higher than the solubility of the molecular encapsulating agent in that embodiment at the reactor temperature. Also contemplated are batch embodiments that are performed at more than one temperature (for example, in which temperature may be raised or lowered in steps or may be raised or lowered gradually); in such embodiments, the concentration of the molecular encapsulating agent is higher than the solubility of the molecular encapsulating agent in that embodiment at one or more of the temperatures used in that embodiment. In some batch embodiments that use more than one temperature, the concentration of the molecular encapsulating agent is higher than the solubility of the molecular encapsulating agent in that embodiment at all of the temperatures used in that embodiment.

In some embodiments, the ratio of the concentration of molecular encapsulating agent to the solubility of the molecular encapsulating agent is 1.1 or higher; or 1.5 or higher; or 2 or higher; or 3 or higher; or 4 or higher. If more than one molecular encapsulating agent is used, it is contemplated that one or more molecular encapsulating agent will be used at a concentration higher than the solubility of that molecular encapsulating agent in that embodiment.

In some batch embodiments of the present invention (known herein as "subsequent cyclopropene" embodiments), all the solvent and all the molecular encapsulating agent are added to the reactor prior to the introduction of cyclopropene. In some of such embodiments, the mixture of solvent and molecular encapsulating agent will contain some dissolved molecular encapsulating agent and will also contain molecular encapsulating agent in the form of a slurry or a supersaturated solution or a combination thereof. In some of such embodiments, the mixture of solvent and molecular encapsulating agent will contain some dissolved molecular encapsulating agent and will also contain molecular encapsulating agent in the form of a slurry. In some embodiments involving molecular encapsulating agent in the form of a slurry, it is contemplated that the contents of the reactor will be agitated, for example by mechanical stirring.

In subsequent cyclopropene embodiments, the cyclopropene may be introduced to the mixture of molecular encapsulating agent and solvent by any method. In some subsequent cyclopropene embodiments, one or more cyclopropene is a gas at the temperature at which the method of making cyclopropene complex is conducted, and the gaseous cyclopropene is introduced by a process in which gaseous cyclopropene is forced by excess pressure directly into the reactor, either above the surface of the mixture of solvent and molecular encapsulating agent or below the surface of that mixture. In some of such embodiments, the gaseous cyclopropene is introduced into the reactor below the surface of the mixture of solvent and molecular encapsulating agent. Introduction of gaseous cyclopropene below the surface of the mixture of solvent and molecular encapsulating agent is known herein as "bubbling."

In some embodiments that involve bubbling, when cyclopropene is first introduced, no bubbles reach the surface of the mixture. It is envisioned that, when cyclopropene is first introduced, molecular encapsulating agent is available to form cyclopropene complex with the cyclopropene and that the cyclopropene forms cyclopropene complex instead of continuing to the surface of the mixture in the form of bubbles. It is further envisioned that, when all of the available molecular encapsulating agent has been incorporated into cyclopropene complexes, then the cyclopropene forms bubbles in the mixture, which rise to the surface and visibly break on the surface. In some subsequent cyclopropene embodiments that involve bubbling, the introduction of cyclopropene is halted when bubbles begin to appear on the surface of the mixture. It is also contemplated that, in such embodiments, some amount of molecular encapsulating agent remains dissolved in the solvent without participating in cyclopropene complex, as determined by the equilibrium between the various ingredients.

Batch methods of the present invention are performed in a reactor that is not sealed. As understood herein, a reactor that is not sealed is a reactor that allows material to escape from the reactor when pressure inside the reactor is above ambient pressure of one atmosphere. In some embodiments, a batch method of the present invention is performed in a reactor with liquid on the bottom and gas (referred to herein as "headspace") on top, and the reactor is constructed so that, whenever pressure inside the reactor is greater than ambient, gas from the headspace is allowed to escape from the reactor. In some of such embodiments, the reactor is constructed so that free passage of gas, unimpeded by valves, caps, or other impediments, is allowed between the headspace and the ambient atmosphere.

Practice of batch embodiments of the present invention gives several benefits. The use of unsealed reactor is simpler than methods using sealed containers (e.g., seals do not have to maintained). The use of ratio of molecular encapsulating agent to solvent that is higher than the solubility of the molecular encapsulating agent in the solvent can lead to one or more of the following: reduction of the amount of waste water, reduction in the amount of wasted cyclopropene, reduction in the amount of wasted molecular encapsulating agent, and/or reduction in the amount of wasted cyclopropene complex.

These benefits can be illustrated by a hypothetical example. It is illustrative to consider a batch process at a fixed temperature, with ingredients chosen with the following characteristics: the solubility of molecular encapsulating agent is 100 g of molecular encapsulating agent in 900 g of water (the solvent); the solubility of cyclopropene complex is 6 grams of cyclopropene complex in 994 g of water; when equilibrium is established among cyclopropene, dissolved molecular encapsulating agent, and cyclopropene complex, the amount of dissolved molecular encapsulating agent is 1 gram per 999 g of water and the amount of dissolved cyclopropene is 1 gram per 999 g of water.

A comparative batch process (i.e., a batch process that is not an embodiment of the present invention) could be conducted in which, at the start of the comparative batch process, the ratio of molecular encapsulating agent to solvent is the same as the solubility of the molecular encapsulating agent in the solvent at the temperature of the batch process. The comparative batch process could be conducted with 10 kg of molecular encapsulating agent and 90 kg of solvent. The amount of cyclopropene could be chosen to have a 1:1 mole ratio with the amount of molecular encapsulating agent. At the conclusion of the comparative batch process, most of the molecular encapsulating agent would have been converted to cyclopropene complex, and most of the complex would be insoluble in the solvent and would be removed by filtration. The following would remain dissolved in the solvent: 90 g of cyclopropene and 540 g of cyclopropene complex and 90 g of molecular encapsulating agent. The solvent (all 90 kg) would be discarded or treated as waste, along with the materials dissolved in the solvent.

The same ingredients could be used in a batch process of the present invention For example, 10 kg of molecular encapsulating agent could be used with 15 kg of solvent. The amount of cyclopropene could be chosen to have a 1:1 mole ratio with the amount of molecular encapsulating agent. At the conclusion of the process, the solvent (now only 15 kg) would contain 15 g of the cyclopropene and 90 g of dissolved cyclopropene complex. Thus, the batch process of the present invention yields reduced waste of solvent, cyclopropene, molecular encapsulating agent, and cyclopropenes complex. Also, the isolated yield of cyclopropene complex is somewhat higher than in the comparative batch process.

It is to be understood that for purposes of the present specification and claims that the range and ratio limits recited herein can be combined. For example, if ranges of 60 to 120 and 80 to 110 are recited for a particular parameter, it is understood that the ranges of 60 to 110 and 80 to 120 are also contemplated. As a further, independent, example, if a particular parameter is disclosed to have suitable minima of 1, 2, and 3, and if that parameter is disclosed to have suitable maxima of 9 and 10, then all the following ranges are contemplated: 1 to 9, 1 to 10, 2 to 9, 2 to 10, 3 to 9, and 3 to 10.

It is to be understood that for purposes of the present specification and claims that each operation disclosed herein is performed at 20°C unless otherwise specified.

### EXAMPLES

### Example 1: Batch Method of Making Cyclopropene Complex

A batch method of making cyclopropene complex was performed as follows:

At room temperature (approximately 23°C), 600 g of commercial alpha-cyclodextrin was suspended in water to make 2400 ml of slurry. While maintaining suspension with mechanical agitation, 250 ml/min of 1-methylcyclopropene was subsurface fed to the reactor. After 70 minutes the reaction was judged finished. An aliquot of about 500 ml was filtered, washed with acetone, and air dried, yielding 120.4 g of 1-methylcyclopropene / alpha-cyclodextrin complex, equivalent to a 578 g recovery.

### Example 2: Batch Method of Making Cyclopropene Complex

A batch method of making cyclopropene complex was performed as follows:

At room temperature (approximately 23°C), 1000 g of commercial alpha-cyclodextrin was suspended in water to make 2400 ml of slurry. While maintaining suspension with mechanical agitation, 250 ml/min of 1-methylcyclopropene was subsurface fed to the reactor. After 90 minutes the reaction was judged finished. An aliquot of about 300 ml was filtered, washed with acetone, and air dried, yielding 132 g of 1-methylcyclopropene / alpha-cyclodextrin complex, equivalent to a 1056 g recovery.

## Claims

1. A batch method for making cyclopropene complex comprising
(a) forming a mixture in a vessel, said mixture comprising
(i) one or more molecular encapsulating agent and
(ii) solvent,wherein the ratio of the amount of said (i) to the amount of said (ii) is higher than the solubility of said molecular encapsulating agent in said solvent at the temperature at which said method is performed, and
(b) introducing one or more cyclopropene into said mixture to form said cyclopropene complex, wherein said cyclopropene has a boiling point of 25°C or lower at one atmosphere pressure, and
(c) after said step (a) and said step (b), removing some or all of said cyclopropene complex from said vessel,wherein said vessel is not sealed while said method is being performed.

2. The method of claim 1, wherein said cyclopropene is gaseous, and wherein said cyclopropene is introduced into said vessel by bubbling.

3. The method of claim 1, wherein said solvent is aqueous.

4. The method of claim 1, wherein said molecular encapsulating agent comprises one or more cyclodextrin.

5. The method of claim 1, wherein said cyclopropene comprises 1-methylcyclopropene and said molecular encapsulating agent comprises alpha-cyclodextrin.

## Patentansprüche

1. Chargenverfahren zur Herstellung eines Cyclopropen-Komplexes, umfassend
(a) das Bilden eines Gemisches in einem Behälter, wobei das Gemisch
(i) ein oder mehrere molekulare Einkapselungsmittel und
(ii) Lösungsmittel umfasst, wobei das Verhältnis der Menge von (i) zu der Menge von (ii) höher ist als die Löslichkeit des molekularen Einkapselungsmittels in dem Lösungsmittel bei der Temperatur, bei der das Verfahren durchgeführt wird, und
(b) das Einbringen eines oder mehrerer Cyclopropene in das Gemisch, um den Cyclopropen-Komplex zu bilden, wobei das Cyclopropen einen Siedepunkt von 25°C oder niedriger bei einem Druck von einer Atmosphäre aufweist, und
(c) nach dem Schritt (a) und dem Schritt (b), das Entfernen von einem Teil oder der Gesamtheit des Cyclopropen-Komplexes aus dem Behälter, wobei der Behälter, während das Verfahren durchgeführt wird, nicht verschlossen ist.

2. Verfahren nach Anspruch 1, wobei das Cyclopropen gasförmig ist, und wobei das Cyclopropen durch Blasenbildung in den Behälter eingeführt wird.

3. Verfahren nach Anspruch 1, wobei das Lösungsmittel wässrig ist.

4. Verfahren nach Anspruch 1, wobei das molekulare Einkapselungsmittel ein oder mehrere Cyclodextrine umfasst.

5. Verfahren nach Anspruch 1, wobei das Cyclopropen 1-Methylcyclopropen uranfasst und das molekulare Einkapselungsmittel Alpha-Cyclodextrin umfasst.

## Revendications

1. Procédé en discontinu de production d'un complexe de cyclopropène comprenant :
a) la formation d'un mélange dans un récipient, ledit mélange comprenant :
(i) un ou plusieurs agents encapsulants moléculaires et
(ii) un solvant, où le rapport de la quantité dudit agent (i) à la quantité dudit solvant (ii) est supérieur à la solubilité dudit agent encapsulant moléculaire dans ledit solvant à la température à laquelle ledit procédé est mis en oeuvre, et
b) l'introduction d'un ou plusieurs cyclopropènes dans ledit mélange pour former ledit complexe de cyclopropène, où ledit cyclopropène a un point d'ébullition inférieur ou égal à 25°C à une pression d'une atmosphère, et
c) après ladite étape (a) et ladite étape (b), l'enlèvement de tout ou partie dudit complexe de cyclopropène dudit récipient, où ledit récipient n'est pas fermé hermétiquement pendant la mise en oeuvre dudit procédé.

2. Procédé selon la revendication 1, où ledit cyclopropène est gazeux et où ledit cyclopropène est introduit dans ledit récipient par barbotage.

3. Procédé selon la revendication 1, où ledit solvant est aqueux.

4. Procédé selon la revendication 1, où ledit agent encapsulant moléculaire comprend une ou plusieurs cyclodextrines.

5. Procédé selon la revendication 1, où ledit cyclopropène comprend du 1-méthylcyclopropène et ledit agent encapsulant moléculaire comprend de l'alpha-cyclodextrine.
